(19) Europäisches Patentamt
European Patent Office
Office européen des brevets

(11) Veröffentlichungsnummer : **0 066 103**
**B1**

(12) # EUROPÄISCHE PATENTSCHRIFT

(45) Veröffentlichungstag der Patentschrift : **28.08.85**

(21) Anmeldenummer : **82103817.1**

(22) Anmeldetag : **04.05.82**

(51) Int. Cl.⁴ : **B 01 J 31/18**, C 07 B 31/00, C 07 C 51/367, C 07 C 85/08, C 07 C 87/28, C 07 C 99/00, C 07 C101/12, C 07 D241/04, C 07 K 1/00

(54) **Verfahren zur Reduktion von reduzierbaren Gruppen und dessen Anwendung.**

(30) Priorität : **29.05.81 DE 3121478**

(43) Veröffentlichungstag der Anmeldung : **08.12.82 Patentblatt 82/49**

(45) Bekanntmachung des Hinweises auf die Patenterteilung : **28.08.85 Patentblatt 85/35**

(84) Benannte Vertragsstaaten : **BE CH DE FR GB IT LI NL**

(56) Entgegenhaltungen :
**DE-A- 2 006 347**
**DE-A- 2 743 031**
**DE-A- 3 012 674**
**US-A- 3 502 571**
**US-A- 4 256 670**

(73) Patentinhaber : **Eckert, Heiner, dr.**
**Lerchenauerstrasse 9**
**D-8000 München 40 (DE)**

(72) Erfinder : **Eckert, Heiner, dr.**
**Lerchenauerstrasse 9**
**D-8000 München 40 (DE)**

(74) Vertreter : **Hansen, Bernd, Dr.rer.nat. et al**
**Hoffmann, Eitle & Partner Patentanwälte Arabellastrasse 4**
**D-8000 München 81 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 066 103 B1

Jouve, 18, rue St-Denis, 75001 Paris, France

**Beschreibung**

Die Erfindung betrifft ein Verfahren zur Reduktion reduzierbarer Gruppen, die ungesättigte C,C-, C,N-, N,N-, N,O-Bindungen, insbesondere $NO_2$-, NO-, NOH-, NR-, CN-, $N_3$-, $N_2$-Gruppen oder C=Cn oder C-Halogen- oder Acylgruppen enthalten, mit Wasserstoff an MPc bzw. mit einem Reduktionsmitel aus der Gruppe $NaBH_4$, $LiAlH_4$ und deren Derivate, und Na/Hg an $[MPc]^{\ominus}$.

In der Literatur werden Verfahren zur Reduktion von z. B. Nitro-Verbindungen, Nitrilen oder C = C-Doppelbindungen beschreiben. Diesbezüglich wird z. B. auf die Ausführungen in der DE-A-30 12 674 hingewiesen, die ein Verfahren zur selektiven Reduktion funktioneller Verbindungen mit einem Reduktionsmittel, wie $H_2$ oder $NaBH_4$ zusammen mit M(Pc), worin M ein Übergangsmetall, insbesondere CO darstellen kann, beschreibt. Ein Hinweis auf die Verwendung von Metallphthalocyaninen mit den Metallen Ru, Os, Rh, Ir, Pd und Pt ist der vorstehend genannten Literaturstelle nicht zu entnehmen.

Übliche heterogene Hydrierkatalysatoren, wie Platinmetalle oder Gerüstkatalysatoren (Raney-Ni, Raney-Co, etc.), wie sie z. B. in « P.N. Rylander : Catalytic Hydrogenation over Platinum Metals », Academic Press, N.Y., 1967, beschrieben sind, haben geringe Selektivität in Reduktionsreaktionen, was auf ihre wenig definierte Struktur zurückzuführen ist. Eine Modifizierung dieser Katalysatoren ist nur auf rein empirischem Wege durch die Einhaltung ganz bestimmter Reaktionsbedingungen bzw. die partielle Vergiftung der Katalysatoren möglich und daher mit grossen Unsicherheiten bezüglich der Durchführung einer bestimmten Zielvorstellung und Reproduzierbarkeit von Ergebnissen verbunden.

Hydrierungen an Metall-Makrozyklen wurden z. B. an (Pyridin) Cobaloxim (II) vorgenommen, wie dies von R. Miyagawa, T. Yamaguchi, in « Nippon Kagaku Kaishi » 1978, S. 160 ff. beschrieben wurde. Die Cobaloxime zeigen jedoch, wie die meisten Vitamin-$B_{12}$-Komplexe, die Tendenz, sich zu zersetzen und neigen zu Nebenreaktionen am Liganden.

US-PS 4 256 670 beschreibt ein Verfahren zur Herstellung von amino-substituierten aromatischen Verbindungen, durch Reduktion der entsprechenden Nitro-Verbindungen bei erhöhter Temperatur und unter erhöhtem Druck. Als Katalysator dienen hierfür Metall-Phthalocyanin-Komplexe, wobei die Metalle der Gruppe VIII des periodischen Systems angehören. Die Reduktionsreaktionen gemäss der vorstehenden Druckschrift werden unter Drücken von 5 bis ca. 5 000 Atmosphären im Autoklaven in Abwesenheit eines Reduktionsmittels durchgeführt. Aufgrund der hohen Temperatur- und Druckbedingungen lässt sich das Verfahren gemäss der vorstehenden Druckschrift labortechnisch schwierig durchführen. Ausserdem ist es unter den genannten Bedingungen nicht möglich, eine Selektivität der Reduktion zu erzielen, d. h. daß durch Wahl der Wertigkeit des im Phthalocyanin-Komplex enthaltenen Metalles sowie durch Einstellung des pH-Wertes eine Reduktion bestimmter Gruppen erfolgt, während andere Gruppen am Molekül intakt bleiben.

Aufgabe der vorliegenden Erfindung ist es, ein Verfahren zur Verfügung zu stellen, welches die Reduktion von ungesättigten C,C-, C,N-, N,N-, N,O-Bindungen sowie von C=C, oder C-Halogen- oder Acylgruppen ermöglicht, insbesondere ist es Aufgabe der Erfindung, ein Verfahren zur Verfügung zu stellen, welches die selektive Reduktion einer bestimmten Gruppe neben anderen reduzierbaren Gruppen ermöglicht.

Diese Aufgabe wird durch ein Verfahren der eingangs genannten Art gelöst, das dadurch gekennzeichnet ist, dass man zur gegebenenfalls selektiven Reduktion ein Platinmetall- Phthalocyanin entsprechend folgender Formel als Katalysator verwendet :

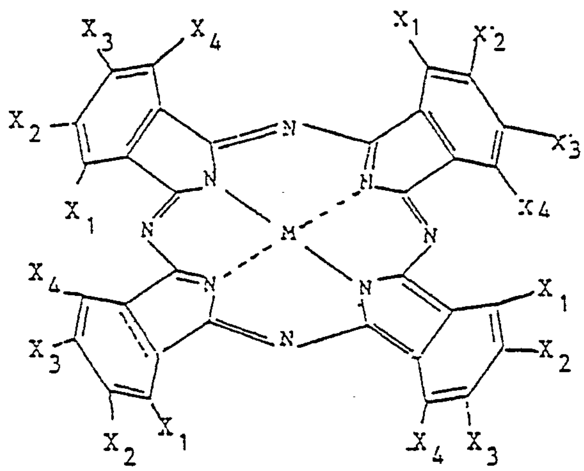

wobei M ein Metall aus der Gruppe Ru, Os, Rh, Ir, Pd und Pt darstellt, $X^{1-4}$ H, Halogen, Cyano oder gegebenenfalls andere Substituenten mit -I-Effekt, Alkyl oder Aryl bedeuten, wobei $X^{2-3}$ auch Bestandteile eines annelierten Ringsystems oder Poly-Pc-Gerüstes sein können, und $X^{1-4}$ unabhängig voneinander einen oder mehrere der benzoiden Ringe substituieren können.

Die erfindungsgemäss verwendeten Platinmetall-Phthalocyanine (MPc) sind thermisch, chemisch und photochemisch extrem stabil und haben im Gegensatz zu üblichen Hydrierkatalysatoren, wie den Platin-Metallen und den Gerüstkatalysatoren eine definierte Struktur. Die MPc bieten deshalb grosse Möglichkeiten zur abgestuften Modifizierung, wie z. B. die Veränderung der Stereochemie am Phthalocyanin-Liganden durch Einführung von Substituenten bzw. polymere Phthalocyanin-Gerüste und/oder Variationen der Metalle M. Ausserdem verlaufen die MPc-katalysierten Reduktionen unter sehr milden Reaktionsbedingungen, worauf nachfolgend noch im Detail eingegangen werden soll. Die erfindungsgemäßen MPc stellen somit einen neuen Hydrierkatalysatortypus dar, welcher die Vorteile der oben aufgeführten beiden Typen (Stabilität bzw. definierte Struktur) vereint, ohne deren Nachteile (schwierige Modifizierbarkeit bzw. Zersetzbarkeit) aufzuweisen.

Gemäss der Erfindung stellt der Katalysator einen Phthalocyanin-Komplex mit einem Metall aus der Reihe Ru, Os, Rh, Ir, Pd und Pt der Platinmetalle dar. Insbesondere ist die Verwendung von Palladium-Phthalocyanin-Katalysatoren bevorzugt.

Als Reduktionsmittel eignet sich bei Verwendung von MPc Wasserstoff bzw. bei Einsatz von $[MPc]^{\ominus}$ $NaBH_4$, $LiAlH_4$ bzw. Na/Hg. Unter den letzteren ist insbesondere die Verwendung von $NaBH_4$ besonders bevorzugt.

MPc-Komplexe werden als bifunktionelle Reaktionskatalysatoren mit steuerbarer Reaktionsspezifität verwendet. Aufgrund ihrer steuerbaren Bifunktionalität sind sie besonders für die selektive Reduktion geeignet. Dies soll anhand der nachfolgenden Gegenüberstellung der Punkte 1) und 2) aufgezeigt werden.

1. Die Reduktion mit Wasserstoff an $M^zPc$ ($z \geq 2$) ist stark pH abhängig und erlaubt eine selektive Reduktion, je nachdem ob die Reaktion im neutralen und sauren Milieu oder im basischen Milieu durchgeführt wird.

A) Im neutralen und sauren Milieu werden reduziert : aromatische Nitroverbindungen, Azomethine, Enamine, aromatische Aldehyde, Olefine, Alkine, Furan, Benzylester. Die Reduktion an Benzylester erfolgt an einer Carbonsäure bevorzugt gegenüber Carbaminsäure, so dass auf diese Weise ein Carbonsäurebenzylester neben einem Carbaminsäurebenzylester selektiv abspaltbar ist.

Nicht reduziert werden bei der vorstehenden Reduktionsreaktion : Aromaten (ausser Furan), aromatische Halogenide, Alkylhalogenide, Acylhalogenide, Carbonsäuren, Carbonsäureester und -amide, Nitrile, aliphatische Aldehyde, Benzylether, -amine und -amide, Alkylcyclopropan.

B) Im basischen Milieu werden reduziert : aromatische Nitroverbindungen, Azomethine, Enamine, Olefine, Alkine, Benzylester, aromatische Halogenide (schnell).

Die Hydrierung von β-Pinen ist stereoselektiv ; es entstehen 71 % cis-Pinan und 29 % trans-Pinan.

Nicht reduziert werden bei dem vorstehenden Verfahren : Aromaten, Alkylhalogenide, Carbonsäuren, Carbonsäureester und -amide, Nitrile, Benzylether, -amine und -amide.

Die pH-abhängige Reaktionsspezifität ist so stark ausgeprägt, dass man bei der Hydrierung an PdPc von p-Chlornitrobenzol bei 20 °C 92 % Anilin erhält, während bei Zusatz von HCl 73 % p-Chloranilin isoliert werden.

2. Die Reduktion mit $NaBH_4$ (oder teilweise auch mit NaHg) an $M^zPc$ ($Z \leq 1$) erfolgt im basischen Milieu ; es werden dabei reduziert :

aromatische und aliphatische Nitroverbindungen, Oxime, Azomethine, Enamine, Nitrile, Olefine, Acetylene, Alkylhalogenide ; ausserdem erfolgt eine reduktive Fragmentierung von fragmentierbaren Verbindungen, wie β-Halogenalkylresten.

Nicht reduziert werden bei diesem Verfahren : Aromaten, aromatische Halogenide, Carbonsäuren, Carbonsäureester und -amide, Cyclopropylcarbonyl-Verbindungen.

$M^zPc$-katalysierte Reduktionen unterscheiden sich in Abhängigkeit von z hauptsächlich dadurch, dass bei $z \geq 2$ insbesondere Alkylhalogenide, Nitrile und aliphatische Nitroverbindungen keine Reduktion erleiden, während Arylhalogenide unter Basenzusatz schnell hydrogenolysiert werden, und bei $z \leq 1$ aliphatische Nitroverbindungen unter Reduktion und Alkylhalogenide sehr schnell unter Hydrogenolyse bzw. Fragmentierung reagieren, während Arylhalogenide nicht reduziert werden.

Die steuerbare Reaktionsspezifität des MPc-Katalysators ist so gross, dass die Reduktionskatalyse von TCBOC-Val-OBz[1]* an $Pd^{II}Pd$ mit $H_2$ nach 1 A) 85 % TCBOC-Val-OH liefert, während an $[Pd^IPc]^{\ominus}$ mit $NaBH_4$ nach 2) 81 % H-Val-OBz[1] entstehen. Im Gegensatz dazu wurde beobachtet, dass der TCBOC-Rest bei der Pd/C-katalysierten Reduktion fast vollständig zersetzt wird (Zersetzungsprodukte > 80 %).

Die Reaktion wird normalerweise unter Schutzgas oder dem Schirm des durch die Reaktion entwickelten Gases durchgeführt. Im übrigen weisen Platinmetall-Phthalocyanine als Katalysatoren gegenüber anderen Hydrierkatalysatoren, wie Raney-Ni oder Palladium auf Kohle, u. a. den Vorteil auf, dass sie an Luft auch im Gemisch mit Wasserstoff nicht entzündlich sind.

Im weiteren müssen sie vor der Reaktion nicht erst hergestellt werden, wie Raney-Ni, und sind — ohne Einhaltung besonderer Lagerbedingungen — unbegrenzt lagerfähig.

Als Solventien kommen alle polaren Lösungsmittel, welche keine reduzierbaren Gruppen tragen, vorzugsweise Alkohole und Alkohol-Wasser-Gemische, insbesondere MeOH, EtOH, tert.-BuOH/$H_2O$ zur Verwendung. Bei den Alkohol-Wasser-Gemischen beträgt das Verhältnis Alkohol : Wasser ca. 10 : 1.

* (TCBOC = 2,2,2-Trichlor-tert.-butyloxycarbonylrest, Val = Valin, OBzl = O-Benzyl)

Die Reaktion wird bei einer Temperatur von 0 bis 200 °C, vorzugsweise von 0 bis 50 °C, und insbesondere bevorzugt bei 20 bis 25 °C, durchgeführt. Insbesondere ist es bevorzugt, bei Verwendung von $H_2$ als Reduktionsmittel in einem Niedrigtemperaturbereich drucklos zu arbeiten.

Die Konzentration des verwendeten Katalysators kann im unteren Bereich 0,001 M betragen. Vorzugsweise wird eine Katalysatorkonzentration von 0,01 bis 0,05 M eingesetzt.

Die Substratkonzentration, d. h. die Konzentration der zu reduzierenden Verbindung kann bis zu 10 M, vorzugsweise 0,1 bis 2 M betragen.

Wie vorstehend ausgeführt, kann die Reduktion durch Zusatz einer freien Base stark beschleunigt werden. Hierfür eignen sich insbesondere primäre, sekundäre oder tertiäre Amine, Phosphine, oder Arsine, vorzugsweise werden sekundäre oder tertiäre Amine verwendet, wobei Triethylamin besonders bevorzugt ist. Hierbei steigt die Reduktionsgeschwindigkeit im allgemeinen um einen Faktor $\geqslant 2$ und bei der Enthalogenierung von Arylhalogeniden um den Faktor $\geqslant 200$ an.

Die nachfolgende Tabelle 1 gibt eine Übersicht über die Selektivität der mit dem Palladium-Phthalocyanin-Komplex katalysierten Reduktionen mit Wasserstoff. Dabei gibt die Tabelle auch die pH-Abhängigkeit der Reaktionen wieder, wobei die Reduktion im ersteren Falle in einem annähernd neutralen Medium, und im zweiten Falle unter Zusatz einer tertiären Base durchgeführt wurde.

Das erfindungsgemässe Verfahren eignet sich insbesondere zur Durchführung einer selektiven Reduktionsreaktion in Gegenwart

1. einer Säurehalogenid-Gruppe, wobei letztere bei der Reduktionskatalyse mit $H_2$ nicht reduziert wird ;
2. einer gegebenenfalls geminal polyhalogenierten Alkylhalogenid-Gruppe, wobei letztere bei der Reduktions mit $H_2$ nicht reduziert wird ;
3. einer Benzylamin- bzw. 1-Ferrocenylalkylamino-Gruppe, unter Erhaltung derselben ;
4. einer Benzylether- bzw. 1-Ferrocenylalkylether-Gruppe, unter Erhaltung derselben ;
5. einer Benzylamid- bzw. 1-Ferrocenylalkylamid-Gruppe, unter Erhaltung derselben.

Ausserdem können mit Hilfe des erfindungsgemässen Verfahrens gegebenenfalls auch substituierte Benzyl- bzw. Benzyloxycarbonylgruppen selektiv neben und unter Erhaltung von leicht reduzierbaren Aromaten bzw. Heteroaromaten hydrogenolytisch abgespalten werden. Im übrigen wird unter Anwendung des erfindungsgemässen Verfahrens auch die gegebenenfalls substituierte Benzylestergruppe selektiv neben und unter Erhaltung von gegebenenfalls geminal polyhalogenierten Alkylhalogenid-Gruppen, insbesondere dem 2,2,2-Trichlor-tert.-benzyloxycarbonyl-Rest, hydrogenolysiert.

Das Verfahren gemäss der Erfindung ist auch geeignet, β-Halogenalkylreste, insbesondere den 2,2,2-Trichlor-tert.-butylrest und den 2,2,2-Trichlor-tert.-butyloxycarbonylrest durch reduktive Fragmentierung mit $NaBH_4$ selektiv von Heteroatomen, wie O, N oder S, abzuspalten. Dies trifft ebenfalls zu für die Abspaltung von β-Halogenalkylresten, insbesondere dem 2,2,2-Trichlor-tert.-butylrest und dem 2,2,2-Trichlor-tert.-butyloxycarbonylrest, selektiv neben einem Benzylester.

Von besonderer Bedeutung ist es, dass mit Hilfe des erfindungsgemässen Verfahrens bei den MPc-katalysierten Reduktionsreaktionen mit $H_2$ durch geeignete Wahl des pH-Wertes aromatische Halogenide wahlweise durch Basenzusatz entweder schnell hydrogenolysiert werden oder durch Säurezusatz keiner Reduktion unterliegen, ohen dass dadurch die Reduktionsreaktion einer anderen funktionellen Gruppe nennenswert beeinträchtigt würde.

(Siehe die Tabelle 1, Seite 5 f.)

Tabelle 1

Selektivität der PdPc-katalysierten Reduktionen mit $H_2$ von funktionellen Gruppen (in Abhängigkeit von pH-Wert)

| Funktionelle Gruppe | Produkt nach Umsetzung mit $H_2$/PdPc | $H_2$/PdPc/tert.Base |
|---|---|---|
| $NO_2$ aromatisch | $NH_2$ | $NH_2$ |
| $NO_2$ aliphatisch | $NO_2$ | - |
| C≡N | C≡N | C≡N |
| C=N | CH-NH | CH-NH |
| CH=O aliphatisch | CH=O | - |
| CH=O aromatisch | $CH_3$ 1) | - |
| C=O aromatisch | C=O | - |
| $CO_2R$ aromatisch, aliphatisch | $CO_2R$ | $CO_2R$ |
| $CONR_2$ aromatisch, aliphatisch | $CONR_2$ | $CONR_2$ |
| COCl aromatisch, aliphatisch | COCl | - |
| C=C konjugiert, isoliert | CH-CH | CH-CH |
| Hal aromatisch | Hal | H |
| Hal aliphatisch | Hal | Hal 2) |
| $CO_2$-Benzyl | $CO_2H$ | $CO_2^{\ominus}$ |
| CON-Benzyl | CON-Benzyl | CON-Benzyl |
| O-Benzyl | O-Benzyl | O-Benzyl |
| N-Benzyl | N-Benzyl | N-Benzyl |
| C=O (aromatisch, aliphatisch) $+NH_2-R$ R = H, Alkyl | CH-NH-R | CH-NH-R |
| 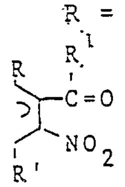 + 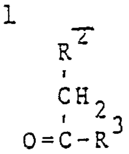 | 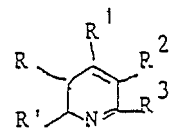 3) | |

Anmerkung zu Tabelle 1

1) langsam, 2) bzw. Alkylierungsprodukt 3) R und R' bilden zusammen ein beliebiges aromatisches System. Die sich am aromatischen System befindliche $R^1C$ = O-Gruppe und die $NO_2$-Gruppe können in beliebiger Stellung zueinander stehen, z. B. in ortho- oder meta-Stellung. $R^1$ und $R^2$ und $R^3$ bedeuten dabei H, Alkyl, Aryl, etc... Bei $R^2$ = Alkyl oder Aryl kann zur Aktivierung einer Methylengruppe eine zusätzliche Carbonylgruppe vorhanden sein. Durch Kondensation wird je nach den Ausgangsverbindungen ein entsprechendes mehrgliedriges heterocyclisches Ringsystem gebildet.

Das erfindungsgemässe Verfahren zur gegebenenfalls selektiven Reduktion unter Verwendung eines Platinmetall-Phthalocyanin-Katalysators eignet sich insbesondere zur Synthese von α-Phenylalkylaminen, wobei von leicht zugänglichen aromatischen Ketonen und Ammoniak bzw. Alkylaminen ausgegangen werden kann. Ausserdem findet das Reduktionsverfahren Anwendung zur Synthese von Benzylalkylaminen, ausgehend von leicht zugänglichen aromatischen Aldehyden und Alkylaminen.

Besondere Anwendung findet das erfindungsgemässe Reduktionsverfahren zur Synthese von N-Alkylaminocarbonsäuren, insbesondere N-Alkyl-α-aminocarbonsäuren, ausgehend von leicht zugänglichen α-Ketocarbonsäuren und Alkylaminen. Es eignet sich darüber hinaus zur Synthese von α-Hydroxycarbonsäuren, wobei als Ausgangsprodukte leicht zugängliche α-Ketocarbonsäuren dienen.

Spezielle Anwendung findet das erfindungsgemässe Reduktionsverfahren zur Synthese von Peptiden, wobei von N-terminal und C-terminal-geschützten Aminosäuren bzw. Oligo- und Polypeptiden ausgegangen wird. Als N-terminale Schutzgruppe ist besonders der 2,2,2-Trichlor-tert.-butyloxycarbonylrest geeignet. Die C-terminale Schutzgruppe stellt bevorzugt einen Benzylrest dar.

Bevorzugte Anwendung findet das erfindungsgemässe Verfahren dadurch, dass

1. die gegebenenfalls auch substituierten Benzyl- bzw. Benzyl-oxycarbonylgruppen von Peptiden, welche aromatische Gruppen enthalten, wie insbesondere die Aminosäuren Histidin, Tryptophan, Tyrosin, Phenylglycin, p-Hydroxyphenylglycin oder Phenylalanin, hydrogenolytisch abgespalten werden ;

2. die Aminosäuren bzw. Oligo- und Polypeptide an Aminofunktionen durch 2,2,2-Trichlor-tert.-butyloxycarbonylreste und an Carboxylfunktionen durch Benzylreste geschützt sind ;

3. bei der selektiven Abspaltung von β-Halogenalkylresten, insbesondere dem 2,2,2-Trichlor-tert.-butyloxycarbonylrest, mit $NaBH_4$ an $[Pd^IPc]^{\ominus}$ bzw. von gegebenenfalls substituierten Benzyl- oder Benzyloxycarbonylresten mit $H_2$ an $Pd^{II}Pc$, sekundäre bzw. tertiäre 1-Ferrocenylalkylamidgruppen erhalten bleiben.

Ausserdem findet das erfindungsgemässe Verfahren bevorzugt Anwendung zur Darstellung des Hexapeptides His-His-Trp-His-Trp-His, zur Synthese von N-Heterocyclen, ausgehend von leicht zugänglichen primären Aminen und Dialdehyden bzw. Diketonen, und zur Synthese von N-mono-substituierten Alkylpiperazinen, ausgehend von leicht zugänglichen primären Aminen und 3-Azaglutardialdehyd, dessen Aminofunktion reversibel geschützt ist, vorzugsweise durch einen 2,2,2-Trichlor-tert.-butyloxycarbonylrest.

Darüber hinaus eignet sich das erfindungsgemässe Reduktionsverfahren zur Synthese von Heterocyclen, wobei als Ausgangsstoffe leicht zugängliche Nitro- bzw. Nitrosoverbindungen dienen können.

Das erfindungsgemässe Verfahren ist besonderes geeignet zur Synthese von Arzneimitteln, Herbiziden und Insektiziden, sowie deren Zwischenprodukten, in denen neben Aminogruppierungen leicht reduzierbare Gruppen, insbesondere aromatisches Halogen bzw. Formyl vorliegen.

## Ausführungsbeispiele 1 bis 65

a) Allgemeine Arbeitsvorschrift zur Platinmetall-Phthalocyanin-katalysierten Reduktion mit Wasserstoff.

10 mMol Edukt bzw. Edukte werden in 5 ml Solvens gelöst und mit 0,1 mMol MPc (PdPc = 60 mg) und gegebenenfalls Zusätzen kräftig gerührt oder geschüttelt. Bei nicht vollständiger Löslichkeit werden bis zu 20 ml Solvens verwendet, bzw. es wird in Suspension gearbeitet (Versuche Nr. 4 bis 9, 24, 26, 30, 31, 49 bis 63).

Nach Sättigen des Reaktionsgemisches mit $H_2$ wird bis zur beendeten bzw. stark nachlassenden Wasserstoffaufnahme reagieren gelassen. Bezüglich der Reaktionszeiten wird auf die nachfolgenden Tabellen verwiesen. Danach entfernt man den Katalysator aus den Reaktionsgemischen, welche das Produkt als Salz enthalten, durch Abfiltrieren und Auswaschen mit einem geeigneten Solvens bzw. Solvensgemisch, wie $EtOH/H_2O$ 1 : 1. Aus den Reaktionsgemischen, welche das neutrale, saure oder basische Produkt nicht salzartig enthalten, gewinnt man den Katalysator durch Abziehen des polaren Solvens (meist ein flüchtiger Alkohol, wie EtOH). Die Aufnahme des Rückstandes erfolgt in einem möglichst unpolaren Solvens oder Solvensgemisch, wie Ether. Darauf erfolgt Abfiltrieren über eine 2 bis 5 cm dicke $Na_2SO_4$-Schicht, welche den Katalysator quantitativ zurückhält und zugleich die Lösung von Wasser, wie Reaktionswasser, befreit. Lösungen, welche stark basische Produkte, wie aliphatische Amine, enthalten, filtriert man unter Inertgas, wie z. B. Stickstoff. Aus dem Filterrückstand gewinnt man den Katalysator durch Herauslösen des $Na_2SO_4$ mit $H_2O$, Waschen mit $H_2O$ und Trocknen bei 20 bis 200 °C quantitativ zurück.

Liegen die Produkte als gelöste Salze vor, so werden sie aus dem mit Ether gewaschenen Filtrat mit Säure, wie Salzsäure, bzw. Basen, wie z. B. Natronlauge, freigesetzt und mit einem unpolaren Solvens oder Solvensgemisch, wie z. B. Ether, extrahiert. Nach dem Einengen der organischen Phase erhält man die Produkte.

b) Beispiel 55a

800 mg (10 mMol) Brenztraubensäure in 2 ml Methanol werden unter kräftigem Rühren mit einer Lösung aus 560 mg (10 mMol) KOH in 5 ml Methanol tropfenweise versetzt (unter Kühlung) und mit 2 ml (20 mMol) n-Butylamin und 60 mg (0,1 mMol) PdPc versetzt. Man sättigt das Reaktionsgemisch mit $H_2$ und rührt unter Zuleitung von $H_2$ aus einer Gasbürette 136 h bei 40 bis 50 °C. Danach wird das Gemisch eingeengt, der Rückstand in Wasser aufgenommen und vom PdPc abfiltriert. Man wäscht das Filtrat mit Ether, engt ein und trocknet den Rückstand im Vakuum über KOH, wobei 960 mg (66 %) N-n-Butyl-D,L-Alaninkaliumsalz zurückbleiben.

c) Beispiel 55b

1,51 g (10 mMol) o-Nitrobenzaldehyd werden in 5 ml Methanol gelöst und mit 1,2 ml (12 mMol) 10 N

HCl (unter Kühlung), 5 ml Aceton und 60 mg (0,1 mMol) PdPc versetzt. Man sättigt das Reaktionsgemisch mit $H_2$ und rührt unter Zuleitung von $H_2$ aus einer Gasbürette 120 h bei 55-60 °C. Danach wird das PdPc abfiltriert und das Filtrat aus Wasser und Hexan verteilt. Man alkalisiert die Wasserphase mit NaOH, extrahiert dreimal mit Ether, trocknet die Etherphase über KOH und engt ein, wobei 610 mg (43 %) 2-Methylchinolin zurückbleiben.

Ausserdem ist es möglich, die Reaktion mit einer Verbindung durchzuführen, die im aromatischen Ring substituiert einen oder mehrere Substituenten, wie z. B. Halogenid, insbesondere Chlorid, Nitril, Carbonsäureamid, -ester, etc. enthält. Ausserdem kann die RCOR-Verbindung betaständig zur Carbonylgruppe Substituenten, wie Halogenid, Nitril, Carbonsäureamid, -ester, enthalten. Die Kondensation kann mit den genannten Ausgangsverbindungen durchgeführt werden, ohne dass eine Reaktion an den substituierten Gruppen erfolgt.

Ausführungsbeispiele 66 bis 71

Allgemeine Arbeitsvorschrift zur platinmetall-phthalocyanin-katalysierten Reduktion mit $NaBH_4$.

Unter $N_2$ werden 2,7 g (70 mMol) $NaBH_4$ in 50 ml EtOH 0,5 g (ca. 0,9 mMol) MPc gegeben. Die tieffarbige Lösung bzw. Suspension (für $[Pd^IPc]^\ominus$ : schwarz) wird mit 10 mMol Edukt (Tabellen 5 und 6) versetzt und unter Wasserkühlung und Druckausgleich bei 20 bis 25 °C (Zeiten aus Tabellen 5 und 6) gerührt. Man neutralisiert das Gemisch unter Eiskühlung mit 5 N HCl (ca. 10 Minuten bis zum Ende der anfangs heftigen Gasentwicklung ; pH 4-7), zentrifugiert (5 Minuten ; 3 000 UpM) vom tiefblauen Niederschlag ab und wäscht diesen dreimal mit MeOH, wobei der Katalysator MPc zurückbleibt. Zur Wiederverwendung wird der Katalysator noch dreimal mit Wasser gewaschen und getrocknet (zwischen 20 und 200 °C). Das vereinigte Zentrifugat wird eingeengt und der Rückstand auf ein geeignetes Solvenspaar, wie $H_2O$/Ether, verteilt. Bei aliphatischen Aminen wird der Rückstand auf 1 N NaOH/Ether verteilt. Nach dem Einengen der über $Na_2SO_4$ getrockneten organischen Phase erhält man die Produkte bzw. nicht-umgesetzten Edukte. Diese werden bei Neutralstoffen durch Filtrieren einer Lösung in Hexan/(Ether), bei Aminen durch Filtrieren einer salzsauren, wässrigen Lösung und bei Aminosäuren bzw. Peptiden durch Filtrieren der zitronensauren Lösung gereinigt.

(Siehe die Tabellen, Seite 8 ff.)

Tabelle 2

PdPc-katalysierte Hydrierung in EtOH, Bedingungen siehe Ausführungsbeispiel

| Nr. | Edukt | Produkt | Rührzeit (h) | Ausbeute (%) | Bemerkung |
|---|---|---|---|---|---|
| 1 | $C_6H_5-NO_2$ | $C_6H_5-NH_2$ | 21,5 | 92 | --- |
| 2 | " | " | 3,5 | 95 | TEA-Zusatz |
| 3 | $p-CH_3-C_6H_4-NO_2$ | $p-CH_3-C_6H_4-NH_2$ | 7,5 | 91 | -- |
| 4 | $p-Cl-C_6H_4-NO_2$ | $C_6H_5-NH_2$ | 120 | 100 | -- |
| 5 | " | " | 22 | 88 | TEA-Zusatz |
| 6 | " | $p-Cl-C_6H_4-NH_2$ | 36 | 73 | HCl-Zusatz |
| 7 | $m-NO_2-C_6H_4-CO-CH_3$ | $m-NH_2-C_6H_4-CO-CH_3$ | 69 | 100 | -- |
| 8 | $m-NO_2-C_6H_4-CH=CH-CO_2Me$ | $m-NH_2-C_6H_4-CH_2-CH_2-CO_2Me$ | 141 | 92 | -- |
| 9 | $C_6H_5-CH=CH-NO_2$ | $C_6H_5-CH_2-CH_2-NO_2$ | 384 | 100 | -- |
| 10 | $n-C_8H_{17}-NO_2$ | Edukt | 140 | (97) | -- |
| 11 | $C_6H_5-CH_2-CN$ | Edukt | 824 | (97) | -- |
| 12 | " | " | 480 | (100) | TEA-Zusatz |
| 13 | " | $(C_6H_5-CH_2-CH_2)_2NH$ | 20 | 100 | 50 atü, 100°C |

Tabelle 2 (Fortsetzung)

| Nr. | Edukt | Produkt | Rührzeit (h) | Ausbeute (%) | Bemerkung |
|---|---|---|---|---|---|
| 14 | Hexen-1 | Hexan | 4 | – | -- |
| 15 | " | " | 8 | – | HCl-Zusatz |
| 16 | " | " | 2,5 | – | Lm: MeOH |
| 17 | " | " | 6,5 | – | Lm:t-BuOH/$H_2O$ (10:1) |
| 18 | " | " | 20 | – | Lm: Eisessig |
| 19 | " | " | 40 | – | Lm: Aceton |
| 20 | " | " | 120 | – | Lm: Acetonitril |
| 21 | " | " | 2 | – | DCHEA-Zusatz |
| 22 | ß-Pinen | cis-Pinan<br>trans Pinan | 395 | 71<br>29 | TEA-Zusatz |
| 23 | Ölsäure | Stearinsäure | 29 | 99,5 | -- |
| 24 | " | " | 12 | 72 | TEA-Zusatz |
| 25 | $C_6H_5-CH=CH-CO_2H$ | $C_6H_5-CH_2-CH_2-CO_2H$ | 6,5 | 98 | -- |
| 26 | $C_6H_5-CH=CH-CO_2Et$ | $C_6H_5-CH_2-CH_2-CO_2Et$ | 40 | 99 | Lm:Acetonitril |
| 27 | $C_6H_5-C(CH_2-CH_3)=CH_2$ | $C_6H_5-CH(CH_2-CH_3)-CH_3$ | 24 | 85 | -- |

Tabelle 2 (Fortsetzung)

| Nr. | Edukt | Produkt | Rühr-Zeit (h) | Ausbeute (%) | Bemerkung |
|---|---|---|---|---|---|
| 28 | Furan | THF | 10 | - | -- |
| 29 | $c-C_3H_5-CH_2-OH$ | Edukt (Ausgangsprodukt) | 100 | (55) | -- |
| 30 | $m-Cl-C_6H_4-CO_2H$ | $C_6H_5-CO_2H$<br>Edukt | 429 | 50<br>(50) | -- |
| 31 | " | $C_6H_5-CO_2H$ | 22 | 99 | TEA-Zusatz |
| 32 | $o-Cl-C_6H_4-CO_2Et$ | Edukt | 125 | (96) | - |
| 33 | " | $C_6H_5-CO_2H$ | 2,5 | 99 | TEA-Zusatz |
| 34 | $o-Cl-C_6H_4-CO_2-CH_2-CH_2-Br$ | Edukt | 210 | 92 | -- |
| 35 | " | $C_6H_5-CO_2-CH_2-CH_2-Br$ | 112 | 67 | TEA-Zusatz |
| 36 | $n-C_{12}H_{25}-Br$ | Edukt | 287 | 100 | -- |
| 37 | $C_6H_5-CHO$ | $C_6H_5-CH_3$ | 16 | 100 | -- |
| 38 | $C_6H_5-CH=CH-CHO$ | $C_6H_5-CH_2-CH_2-CH_2-OH$ | 33 | 80 | Lm: Acetonitril |
| 39 | $C_6H_5-CH(CH_3)-CHO$ | Edukt | 22 | (100) | Diäthylacetal |
| 40 | $C_6H_5-CH=N-i-C_3H_7$ | $C_6H_5-CH_2-NH-i-C_3H_7$ | 4 | 100 | -- |
| 41 | $C_6H_5-CH(CH_3)-CH=N-n-C_4H_9$ | $C_6H_5-CH(CH_3)-CH_2-NH-C_4H_9$ | 27 | 100 | -- |

Tabelle 2 (Fortsetzung)

| Nr. | Edukt | Produkt | Rührzeit (h) | Ausbeute (%) | Bemerkung |
|---|---|---|---|---|---|
| 42 | 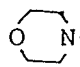-CH=C$(CH_3)_2$ | 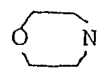-$CH_2$-i-$C_3H_7$ | 4 | 77 | -- |
| 43 | $C_6H_5$-$CH_2$-$CO_2$-$CH_2$-$C_6H_5$ | $C_6H_5$-$CH_2$-$CO_2H$ | 12 | 98 | -- |
| 44 | " | " | 5 | 66 | TEA-Zusatz |
| 45 | n-$C_8H_{17}$-O-$CH_2$-$C_6H_5$ | Edukt | 95 | (99) | -- |
| 46 | " | " | 144 | (100) | TEA-Zusatz |
| 47 | $C_6H_5$-CO-NH-$CH_2$-$C_6H_5$ | " | 110 | (100) | -- |
| 48 | " | " | 266 | (97) | TEA-Zusatz |
| 49 | $C_6H_5$-COCl | " | 260 | (93) | Lm: Acetonitril |
| 50 | $C_6H_5$-CH=CH-COCl | " | 40 | (94) | " " |
| 51 | Ölsäurechlorid | " | 21,5 | (99) | " " |

Abkürzungen : TEA = Triethylamin, DCHEA = Dicyclohexylethylamin, THF = Tetrahydrofuran

Tabelle 3
MPc-katalysierte Hydrierungen in EtOH, Bedingungen siehe Ausführungsbeispiele

| Nr. | M | Edukt | Produkt | Rührzeit (h) | Ausbeute (%) |
|---|---|---|---|---|---|
| 11 | Pd | $C_6H_5-NO_2$ | $C_6H_5-NH_2$ | 21,5 | 92 |
| 52 | Pt | $C_6H_5-NO_2$ | $C_6H_5-NH_2$ | 180 | 83 |

Tabelle 4
Eintopfsynthesen von Aminen durch reduktive Aminierung mit $H_2$, PdPc-katalysiert von aromatischen und aliphatischen Carbonylverbindungen, Bedingungen siehe Ausführungsbeispiel

| Nr. | Edukt | Produkt | Rührzeit (h) | Ausbeute (%) |
|---|---|---|---|---|
| 53 | $C_6H_5-CO-CH_3$<br>$NH_3$ | $C_6H_5CH-CH_3$<br>$NH_2$ | 307 | 52 |
| 54 | $C_6H_5-CH(CH_3)-CHO$<br>$NH_2-n-C_4H_9$ | $C_6H_5-CH(CH_3)-CH_2NH-n-C_4H_9$ | 28 | 100 |
| 55 | $C_6H_5-CHO$<br>$NH_2-i-C_3H_7$ | $C_6H_5-CH_2-NH-i-C_3H_7$ | 8 | 82 |
| 55a | $CH_3-CO-CO_2H$<br>$n-C_4H_9-NH_2$ | $CH_3$<br>$n-C_4H_9-NH-CH-CO_2H$ | 136 | 66 |
| 55b | 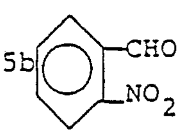 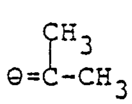 | 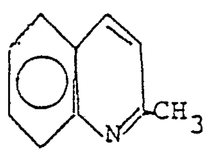 | 120 | 43 |

(Siehe die Tabelle 5 , Seite 13 f.)

Tabelle 5

PdPc als bifunktioneller Reduktionskatalysator mit steuerbarer Reaktionsspezifität bei der selektiven Abspaltung von Schutzgruppen in der Peptidchemie, Bedingungen siehe Ausführungsbeispiel

| Nr. | Red.-Mittel | Solvens | Edukt [1)] | Produkt | Rührzeit (h) | Ausbeute (%) |
|---|---|---|---|---|---|---|
| 56 | $H_2$ | EtOH | BEOC-Val-OBzl [2)] | BEOC-Val-OH | 22 | 56 |
| 57 | " | " | TCBOC-Val-OMe | Edukt | 20 | (97) |
| 58 | " | " | Z-Ala-Val-OBzl | Z-Ala-Val-OH | 75 | 72 |
| 59 | " | t-BuOH/$H_2O$ | TCBOC-Val-OBzl | TCBOC-Val-OH | 46 | 85 |
| 60 | " | (10:1) | TCBOC-Aib-Ala-OBzl | TCBOC-Aib-Ala-OH | 6 | 92 |
| 61 | " | " | TCBOC-Val-Aib-OBzl | TCBOC-Val-Aib-OH | 135 | 89 |
| 62 | " | " | TCBOC-Aib-Pro-OBzl | TCBOC-Aib-Pro-OH | 44 | 67 |
| 63 | " | " | TCBOC-Gly-Leu-OBzl | TCBOC-Gly-Leu-OH | 68 | 73 |
| 64 | " | Essigsäure | BOC-Gly-Leu-N-<br>-N-Val-NH-$CH_2$-Fc<br>$CH_2$-FC | Edukt | 70 | (89) zurückgewonnenes Ausgangsprodukt |
| 65 | " | EtOH | " | " | 70 | (98) |
| 66 | $NaBH_4$ | " | " | " | 30 | (1oo) |
| 67 | " | " | TCBOC-Phe-OtBu | H-Phe-OtBu | 18 | 82 |
| 68 | " | " | TCBOC-Val-OBzl | H-Val-OBzl | 20 | 81 |

1) in der Peptidchemie übliche Abk. nach « E. Wünsch, Houben-Weyl Bd. 15, Thieme, Stuttgart, 1974. TCBOC = 2,2,2-Trichlor-tert.-loxycarbonyl ;
FC = Ferrocenyl

2) OBzl = O-Benzyl

Tabelle 6
PdPc-katalysiertte Reduktion mit $NaBH_4$ in EtOH, Bedingungen siehe Ausführungsbeispiele

| Nr. | Edukt | -Produkt | Rührzeit | Ausbeute |
|---|---|---|---|---|
| 69 | $p-Cl-C_6H_5NO_2$ | $p-Cl-C_6H_5NH_2$ | 2 | 80 |
| 70 | " | " | 120 | 98 |
| 71 | $C_6H_5CH=CHCO_2Et$ | $C_6H_5CH_2CH_2CO_2Et$ | 0,5 | 93 |

**Patentansprüche**

1. Verfahren zur Reduktion reduzierbarer Gruppen, die ungesättigte C,C-, C,N-, N,N-, N,O-Bindungen, insbesondere $NO_2$, NO, NOH, NR, CN, $N_3$, $N_2$-Gruppen oder C=C, oder C-Halogen- oder Acylgruppen enthalten, dadurch gekennzeichnet, daß man zur gegebenenfalls selektiven Reduktion a) mit Wasserstoff an Metall-Phthalocyanin (MPc) oder b) mit einem Reduktionsmittel aus der Gruppe $NaBH_4$, $LiAlH_4$ und deren Derivate, und Na/Hg, an $[MPc]^{\ominus}$, ein Platinmetall-Phthalocyanin entsprechend folgender Formel als Katalysator verwendet :

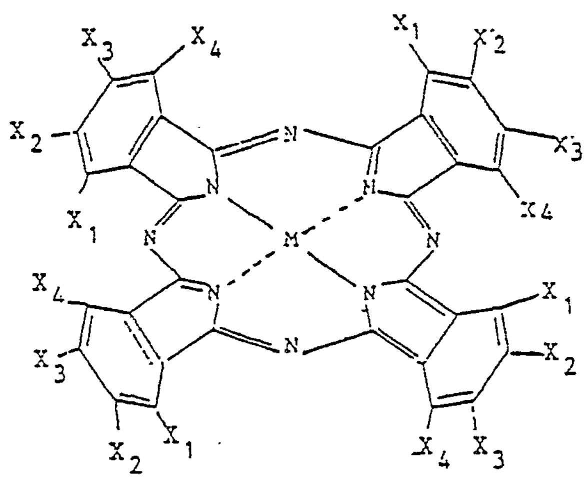

wobei M ein Metall aus der Gruppe Ru, Os, Rh, Ir, Pd und Pt darstellt, $X^{1\text{-}4}$H, Halogen, Cyano oder gegebenenfalls andere Substituenten mit -I-Effekt, Alkyl oder Aryl bedeuten, wobei $X^{2\text{-}3}$ auch Bestandteile eines annellierten Ringsystems oder Poly-Pc-Gerüstes sein können, und $X^{1\text{-}4}$ unabhängig voneinander einen oder mehrere der benzoiden Ringe substituieren können.

2. Verfahren gemäss Anspruch 1, dadurch gekennzeichnet, dass die Änderung der Oxidationsstufe z des Metalls M von $Z \geqslant 2$ nach $z \leqslant 1$ beträgt.

3. Verfahren gemäss Ansprüchen 1 bis 2, dadurch gekennzeichnet, dass $H_2$ als Reduktionsmittel drucklos eingesetzt wird.

4. Verfahren nach einem oder mehreren der vorangehenden Ansprüche, dadurch gekennzeichnet, dass die Reduktion durch Zusatz einer freien Base stark beschleunigt wird.

5. Verfahren nach einem oder mehreren der vorangehenden Ansprüche, dadurch gekennzeichnet, dass die Reduktionsreaktion selektiv in Gegenwart einer Säurehalogenid-Gruppe durchgeführt wird, wobei letztere bei der Reduktionskatalyse mit $H_2$ intakt bleibt.

6. Verfahren nach einem oder mehreren der Ansprüche 1 bis 4, dadurch gekennzeichnet, dass die Reduktionskatalyse selektiv in Gegenwart einer gegebenenfalls geminal polyhalogenierten Alkylhalogenid-Gruppe durchgeführt wird, wobei letztere bei der Reduktionsreaktion mit $H_2$ intakt bleibt.

7. Verfahren nach einem oder mehreren der Ansprüche 1 bis 4, dadurch gekennzeichnet, dass die Reduktionsreaktion selektiv in Gegenwart einer Benzylamin- bzw. 1-Ferrocenylalkylamino-gruppe durchgeführt wird, wobei letztere intakt bleiben.

8. Verfahren nach einem oder mehreren der Ansprüche 1 bis 4, dadurch gekennzeichnet, dass die Reduktionskatalyse selektiv in Gegenwart einer Benzylether- bzw. 1-Ferrocenylalkylethergruppe durchgeführt wird, wobei letztere intakt bleiben.

9. Verfahren nach einem oder mehreren der Ansprüche 1 bis 4, dadurch gekennzeichnet, dass die Reduktionskatalyse selektiv in Gegenwart einer Benzylamid- bzw. 1-Ferrocenylalkylamidgruppe durchgeführt wird, wobei letztere intakt bleiben.

10. Verfahren nach einem oder mehreren der vorangehenden Ansprüche, dadurch gekennzeichnet, dass die gegebenenfalls auch substituierten Benzyl- bzw. Benzyloxycarbonylgruppen selektiv neben und unter Erhaltung von leicht reduzierbaren Aromaten bzw. Heteroaromaten hydrogenolytisch abgespalten werden.

11. Verfahren nach einem oder mehreren der vorangehenden Ansprüche, dadurch gekennzeichnet, dass die gegebenenfalls auch substituierte Benzylestergruppe selektiv neben und unter Erhaltung von gegebenenfalls geminal polyhalogenierten Alkylahalogenid-Gruppen, insbesondere dem 2,2,2-Trichlor-tert.-butyloxycarbonyl-Rest, hydrogenolysiert wird.

12. Verfahren nach einem oder mehreren der vorangehenden Ansprüche, dadurch gekennzeichnet, dass β-Halogenalkylreste, insbesondere der 2,2,2-Trichlor-tert.-butylrest und der 2,2,2-Trichlor-tert.-butyloxycarbonylrest durch reduktive Fragmentierung mit $NaBH_4$ selektiv von Heteroatomen, wie O, N oder S, abgespalten werden.

13. Verfahren nach einem oder mehreren der vorangehenden Ansprüche, dadurch gekennzeichnet, dass β-Halogenalkylreste, insbesondere der 2,2,2-Trichlor-tert.-butylrest und der 2,2,2-Trichlor-tert.-butyloxycarbonylrest selektiv neben einem Benzylester abgespalten werden.

14. Verfahren nach einem oder mehreren der vorangehenden Ansprüche, dadurch gekennzeichnet, dass bei den MPc-katalysierten Reduktionsreaktionen mit $H_2$ durch geeignete Wahl des pH-Wertes aromatische Halogenide wahlweise durch Basenzusatz entweder schnell hydrogenolysiert werden oder durch Säurezusatz intakt bleiben, ohne dass dadurch die Reduktionsreaktion einer anderen funktionellen Gruppe nennenswert beeinträchtigt würde.

15. Anwendung des Verfahrens gemäss einem oder mehreren der vorangehenden Ansprüche zur Synthese von α-Phenylalkylaminen, ausgehend von leicht zugänglichen aromatischen Ketonen und Ammoniak bzw. Alkylaminen.

16. Anwendung des Verfahrens gemäss einem oder mehreren der Ansprüche 1 bis 14 zur Synthese von Benzylalkylaminen, ausgehend von leicht zugänglichen aromatischen Aldehyden und Alkylaminen.

17. Anwendung des Verfahrens gemäss einem oder mehreren der Ansprüche 1 bis 14 zur Synthese von N-Alkylaminocarbonsäuren, insbesondere N-Alkyl-α-aminocarbonsäuren, ausgehend von leicht zugänglichen α-Ketocarbonsäuren und Alkylaminen.

18. Anwendung des Verfahrens gemäss einem oder mehreren der Ansprüche 1 bis 14 zur Synthese von α-Hydroxycarbonsäuren, ausgehend von leicht zugänglichen α-Ketocarbonsäuren.

19. Anwendung des Verfahrens gemäss einem oder mehreren der Ansprüche 1 bis 14 zur Synthese von Peptiden, ausgehend von leicht zugänglichen geschützten Aminosäuren bzw. Oligo- und Polypeptiden.

20. Anwendung des Verfahrens gemäss Anspruch 10, dadurch gekennzeichnet, dass die gegebenenfalls auch substituierten Benzyl- bzw. Benzyloxycarbonylgruppen von Peptiden, welche aromatische Gruppen enthalten, wie insbesondere die Aminosäuren, Histidin, Tryptophan, Tyrosin, Phenylglycin, p-Hydroxyphenylglycin oder Phenylalanin, hydrogenolytisch abgespalten werden.

21. Anwendung gemäss Anspruch 19, dadurch gekennzeichnet, dass die Aminosäuren bzw. Oligo- und Polypeptide an Aminofunktionen durch 2,2,2-Trichlor-tert.-butyloxycarbonylreste und an Carboxylfunktionen durch Benzylreste geschützt sind.

22. Anwendung gemäss Ansprüchen 19 bis 21 dadurch gekennzeichnet, dass bei der selektiven Abspaltung von β-Halogenalkylresten, insbesondere dem 2,2,2-Trichlor-tert.-butyloxycarbonylrest, mit $NaBH_4$ an $[Pd^IPc]^{\ominus}$ bzw. von gegebenenfalls substituierten Benzyl- oder Benzyloxycarbonylresten mit $H_2$ an $Pd^{II}Pc$, sekundäre bzw. tertiäre 1-Ferrocenylalkylamidgruppen erhalten bleiben.

23. Anwendung des Verfahrens gemäss Ansprüchen 1 bis 14 zur Darstellung des Hexapeptides His-His-Trp-His-Trp-His.

24. Anwendung des Verfahrens gemäss Ansprüchen 1 bis 14 zur Synthese von N-Heterocyclen, ausgehend von leicht zugänglichen primären Aminen und Dialdehyden bzw. Diketonen.

25. Anwendung des Verfahrens gemäss Ansprüchen 1 bis 14 zur Synthese von N-mono-substituierten Alkylpiperazinen, ausgehend von leicht zugänglichen primären Aminen und 3-Azaglutardialdehyd, dessen Aminofunktion reversibel geschützt ist, vorzugsweise durch einen 2,2,2-Trichlor-tert.-butyloxycarbonylrest.

26. Anwendung des Verfahrens gemäss einem oder mehreren der Ansprüche 1 bis 14 zur Synthese von N-Heterocyclen, ausgehend von leicht zugänglichen Nitro- bzw. Nitrosoverbindungen.

27. Anwendung des Verfahrens gemäss einem oder mehreren der Ansprüche 1 bis 14 zur Synthese von Arzneimitteln, Herbiziden und Insektiziden und deren Zwischenprodukten, in denen neben Aminogruppierungen leicht reduzierbare Gruppen, insbesondere aromatisches Halogen bzw. Formyl vorliegen.

**Claims**

1. A process for the reduction of reducible groups which contain unsaturated C,C-, C,N-, N,N-, N,O-

bonds, especially $NO_2$, NO, NOH, NR, CN, $N_3$, $N_2$-groups or C = C, or C-halogen or acyl groups, characterized in that for optionally selective reduction a) with hydrogen on metal-phthalocyanine (MPc) or b) with a reduction agent selected from the group $NaBH_4$, $LiAlH_4$ and its derivatives and Na/Hg, on $[MPc]^-$, a platinum metal-phthalocyanine of the folloxing formula is used as the catalyst :

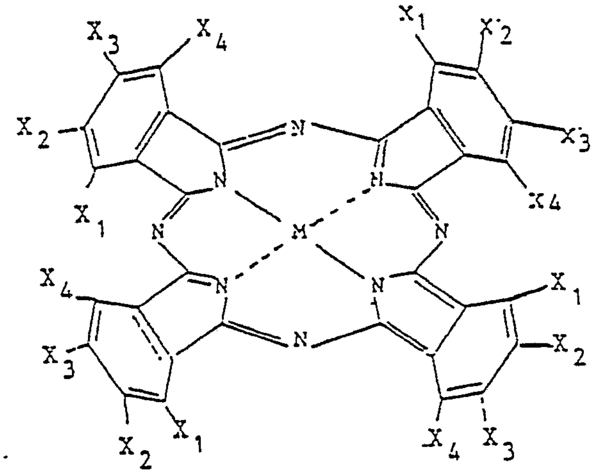

wherein M is a metal selected from the group Ru, Os, Rh, Ir, Pd and Pt, $X^{1-4}$ is hydrogen, halogen, cyano or optionally other substituents with -I-effect, alkyl or aryl, while $X^{2-3}$ can also be components of an annellated ring system or poly-Pc-structure and $X^{1-4}$ can independently substitute one or more of the benzoid rings.

2. A process according to claim 1, characterized in that the change of the oxidation phase z of the metal M is $z \geqslant 2$ to $z \leqslant 1$.

3. A process according to claim 1 or 2, characterized in that $H_2$ is used as a reduction agent without pressure.

4. A process according to one or more of the claims above, characterized in that the reduction is greatly speeded by the addition of a free base.

5. A process according to one or more of the claims above, characterized in that the reduction reaction is performed selectively in the presence of an acid-halogen group, while the latter remains unaffected during the reduction catalysis with $H_2$.

6. A process according to one or more of claims 1 to 4, characterized in that the reduction catalysis is carried out selectively in the presence of an optionally geminal polyhalogenated alkylhalide group whereby the latter remains unaffected during the reduction reaction with $H_2$.

7. A process according to one or more of claims 1 to 4, characterized in that the reduction reaction is selectively carried out in the presence of a benzylamine or 1-ferrocenylalkylamino group whereby the latter remain unaffected.

8. A process according to one or more of claims 1 to 4, characterized in that the reduction catalysis is selectively performed in the presence of a benzyl ether- or 1-ferrocenylalkylether group, while the latter remain unaffected.

9. A process according to one or more of claims 1 to 4, characterized in that the reduction catalysis is selectively performed in the presence of a benzyl amide- or 1-ferocenylalkylamide group, while the latter remain unaffected.

10. A process according to one or more of the above claims, characterized in that the optionally substituted benzyl- or benzyloxycarbonyl groups are selectively hydrogenolytically split off in the presence of and maintaining easily reducible aromatic or heteroaromatic compounds.

11. A process according to one or more of the above claims, characterized in that the optionally substituted benzyl ester group is selectively hydrogenolysed in the presence of and maintaining optionally geminal polyhalogenised alkyl halide groups, especially the 2,2,2-trichloro-tert.-butyloxycarbonyl residue.

12. A process according to one or more of the above claims, characterised in that β-halogenalkyl residues, especially the 2,2,2-trichloro-tert.-butyl residue and the 2,2,2-trichloro-tert.-butyloxycarbonyl residue are split off selectively from heteroatoms, e.g. O, N or S, by reductive fragmentation with $NaBH_4$.

13. A process according to one or more of the above claims, characterized in that β-halogenalkyl residues, especially the 2,2,2-trichloro-tert.-butyl residue ane the 2,2,2-trichloro-tert.-butyloxycarbonyl residue are split off selectively beside a benzyl ester.

14. A process according the one or more of the above claims, characterized in that in the case of MPc-catalyzed reduction reactions with $H_2$ by appropriate selection of the pH value aromatic halides are either quickly hydrogenolized by selective addition of a base, or they remain unaffected by addition of an acid, without markedly influencing the reduction reaction of another functional group.

15. Use of the process according to one or more of the above claims for the synthesis of α-phenylalkyl amines, starting from easily available aromatic ketones and ammonia or alkyl amines.

16. Use of the process according to one or more of claims 1 to 14 for the synthesis of benzylalkyl amines, starting from easily available aromatic aldehydes and alkyl amines.

17. Use of the process according to one or more of claims 1 to 14 for the synthesis of N-alkylaminocarboxylic acids, especially N-alkyl-α-aminocarboxylic acids, starting from easily available α-ketocarboxylic acids and alkyl amines.

18. Use of the process according to one or more of claims 1 to 14 for the synthesis of α-hydroxycarboxylic acids, starting from easily available α-ketocarboxylic acids.

19. Use of the process according to one or more of claims 1 to 14 for the synthesis of peptides, starting from the easily available protected amino acids or oligo- and polypeptides.

20. Use of the process according to claim 10, characterized in that the optionally substituted benzyl or benzyloxycarbonyl groups are hydrogenolytically split off of from peptides which contain aromatic groups such as especially the amino acids histidine, tryptophane, tyrosine, phenyl glycine, p-hydroxyphenylglycine or phenylalanine.

21. Use of the process according to claim 19, characterized in the amino acids or oligo- and polypeptides are protected by 2,2,2-trichloro-tert.-butyloxycarbonyl residues and by benzyl residues at the amino functions and on the carboxyl functions.

22. Use of the process according to claims 19 to 21, characterized in that in the selective splitting off of β-halogenalkyl radicals, especially 2,2,2-trichloro-tert.-butyloxycarbonyl residue, with $NaBH_4$ on $[Pd^IPc]^\ominus$ or of optionally substituted benzyl or benzyloxycarbonyl residues with $H_2$ on $Pd^{II}Pc$, secondary or tertiary 1-ferrocenylalkylamide groups are maintained.

23. Use of the process according to claims 1 to 14, characterized in that the hexapeptide His-His-Trp-His-Trp-His is prepared.

24. Use of the process according to claims 1 to 14 for the synthesis of N-heterocyclic compounds, starting from easily available primary amines and dialdehydes or diketones.

25. Use of the process according to claims 1 to 14 for the synthesis of N-mono-substituted alkyl piperazines, starting from easily available primary amines and 3-azaglutardialdehyde, the amino function of which is reversibly protected, preferably by a 2,2,2-trichloro-tert.-butyloxycarbonyl residue.

26. Use of the process according to one or more of claims 1 to 14 for the synthesis of N-heterocyclic compounds, starting from easily available nitro- or nitroso compounds.

27. Use of the process according to one or more of claims 1 to 14 for the synthesis or pharmaceuticals, herbicides and insecticides and their intermediate products in which apart from the amino groupings, easily reducible groups are present, especially aromatic halogen or formyl.

**Revendications**

1. Procédé pour la réduction de groupes réductibles, qui contiennent des liaisons C,C, C,N, N,N, N,O insaturées, en particulier des groupes $NO_2$, NO, NOH, NR, CN, $N_3$, $N_2$ ou C = C ou des groupes C-halogène ou acyle, caractérisé par le fait que l'on utilise comme catalyseur pour la réduction éventuellement sélective a) avec l'hydrogène en pésence d'une phtalocyanine métallique (MPc) ou b) avec un agent de réduction choisi parmi le groupe comprenant $NaBH_4$, $LiAlH_4$ et leurs dérivés, et Na/Hg, en présence de $(MPc)^\ominus$, une phtalocyanine métallique de la famille du platine correspondant à la formule suivante

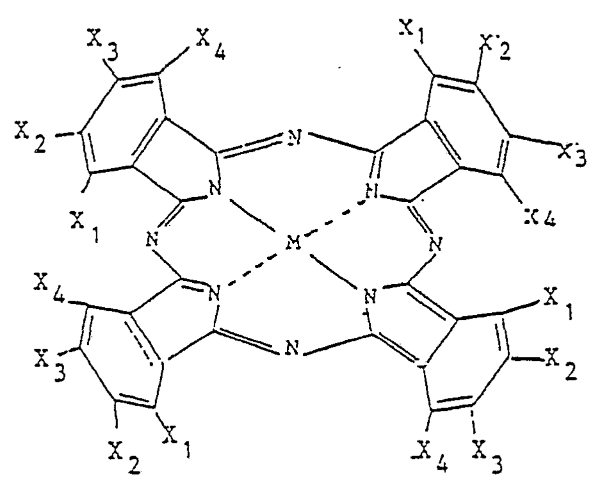

dans laquelle M représente un métal choisi dans le groupe comprenant Ru, Os, Rh, Ir, Pd et Pt ; $X^{1-4}$ représentent H, un halogène, un groupe cyano ou éventuellement d'autres substituants avec effet-I, un

reste alkyle ou aryle, $X^{2\text{-}3}$ pouvant être aussi des éléments d'un système de noyaux condensés ou d'un squelette poly-Pc, et $X^{1\text{-}4}$ indépendamment les uns des autres pouvant être des substituants sur un ou plusieurs noyaux benzéniques.

2. Procédé selon la revendication 1, caractérisé par le fait que le changement de degré d'oxydation z du métal s'élève de $z \geqslant 2$ et $z \leqslant 1$.

3. Procédé selon les revendications 1 et 2, caractérisé par le fait que $H_2$ est utilisé comme agent de réduction sans pression.

4. Procédé selon l'une ou plusieurs des revendications précédentes, caractérisé par le fait que la réduction est fortement accélérée par addition d'une base libre.

5. Procédé selon l'une ou plusieurs des revendications précédentes, caractérisé par le fait que la réaction de réduction est réalisée sélectivement en présence d'un groupe d'halogénure d'acide, ce dernier restant intact lors de la catalyse de la réduction avec $H_2$.

6. Procédé selon l'une ou plusieurs des revendications 1 à 4, caractérisé par le fait que la catalyse de la réduction est réalisée sélectivement en présence d'un groupe halogéno-alkyle éventuellement géminé polyhalogéné, ce dernier restant intact lors de la réaction de réduction avec $H_2$.

7. Procédé selon l'une ou plusieurs des revendications 1 à 4, caractérisé par le fait que la réaction de réduction est réalisée sélectivement en présence d'un groupe benzylamine ou 1-ferrocénylalkylamino, ce dernier restant intact.

8. Procédé selon l'une ou plusieurs des revendications 1 à 4, caractérisé par le fait que la catalyse de la réduction est réalisée sélectivement en présence d'un groupe benzyléther ou 1-ferrocénylalkyléther, ce dernier restant intact.

9. Procédé selon l'une ou plusieurs des revendications 1 à 4, caractérisé par le fait que la catalyse de la réduction est réalisée sélectivement en présence d'un groupe benzylamide ou 1-ferrocénulalkylamide, ce dernier restant intact.

10. Procédé selon l'une ou plusieurs des revendications précédentes, caractérisé par le fait que les groupes benzyle ou benzyloxycarbonyle, éventuellement même substitués, sont séparés par hydrogénolyse sélectivement à côté et lors de l'obtention de carbures aromatiques ou de carbures hétéroaromatiques facilement réductibles.

11. Procédé selon l'un ou plusieurs des revendications précédentes, caractérisé par le fait que le groupe benzylester, éventuellement même substitué, est hydrogénolysé sélectivement à côté et lors de l'obtention de groupes halogéno-alkyle éventuellement géminés polyhalogénés, en particulier du reste 2,2,2-trichloro-tert-butyloxycarbonyle.

12. Procédé selon l'une ou plusieurs des revendications précédentes, caractérisé par le fait que les restes β-halogénoalkyle, en particulier le reste 2,2,2-trichloro-tert-butyle et le reste 2,2,2-trichloro-tert-butyloxycarbonyle, sont séparés en fragmentant par voie réductrice avec $NaBH_4$ sélectivement les hétéroatomes comme O, N ou S.

13. Procédé selon l'une ou plusieurs des revendications précédentes, caractérisé par le fait que les restes β-halogénoalkyle, en particulier le reste 2,2,2-trichloro-tert-butyle et le reste 2,2,2-trichloro-tert-butyloxycarbonyle sont séparés sélectivement à côté d'un ester benzylique.

14. Procédé selon l'une ou plusieurs des revendications précédentes, caractérisé par le fait que, lors des réactions de réduction avec $H_2$ catalysées par MPc, grâce au choix approprié de la valeur du pH, les halogénures aromatiques, selon nécessité, soit sont rapidement hydrogénolysés par addition d'une base soit restent intacts par addition d'un acide, sans que cela n'influence défavorablement de manière notable la réaction de réduction d'un autre groupe fonctionnel.

15. Utilisation du procédé selon l'une ou plusieurs des revendications précédentes pour la synthèse des α-phénylalkylamines, en partant de cétones aromatiques facilement accessibles et d'ammoniac ou d'alkylamines.

16. Utilisation du procédé selon l'une ou plusieurs des revendications 1 à 14, pour la synthèse des benzylalkylamines, en partant d'aldéhydes aromatiques facilement accessibles et d'alkylamines.

17. Utilisation du procédé selon l'une ou plusieurs des revendications 1 à 14, pour la synthèse des acides N-alkylaminocarboxyliques, en particulier des acides N-alkyl-α-aminocarboxyliques, en partant d'acides α-cétocarboxyliques facilement accessibles et d'alkylamines.

18. Utilisation du procédé selon l'une ou plusieurs des revendications 1 à 14, pour la synthèse des acides α-hydroxycarboxyliques, en partant d'acides α-cétocarboxyliques facilement accessibles.

19. Utilisation du procédé selon l'une ou plusieurs des revendications 1 à 14, pour la synthèse des peptides, en partant d'aminoacides protégés ou d'oligo- et polypeptides, facilement accessibles.

20. Utilisation du procédé selon la revendication 10, caractérisé par le fait que les groupes benzyle ou benzyloxycarbonyle éventuellement même substitués des peptides, qui contiennent des groupes aromatiques, comme en particulier les aminoacides, l'histidine, le tryptophane, la tyrosine, la phénylglycine, la p-hydroxyphénylglycine ou la phénylalamine, sont séparés par hydrogénolyse.

21. Utilisation selon la revendication 19, caractérisée par le fait que les aminoacides ou les oligo- et polypeptides sont protégés sur les fonctions amines par le reste 2,2,2-trichloro-tert-butyloxycarbonyle et sur les fonctions carboxyle par le reste benzyle.

22. Utilisation selon les revendications 19 à 21, caractérisée par le fait que, lors de la séparation sélective des restes β-halogénoalkyle, en particulier du reste 2,2,2-trichloro-tert-butyloxycarbonyle, avec

$HaBH_4$ en présence de $(Pd^{I}Pc)^{\ominus}$ ou des restes benzyle ou benzyloxycarbonyle, éventuellement substitués, avec $H_2$ en présence de $Pd^{II}Pc$, les groupes 1-ferrocénylalkylamide secondaires ou tertiaires restent intacts.

23. Utilisation du procédé selon les revendications 1 à 14, pour l'obtention de l'hexapeptide His-His-Trp-His-Trp-His.

24. Utilisation du procédé selon les revendications 1 à 14, pour la synthèse de composés N-hétérocycliques, en partant d'amines primaires et de dialdéhydes ou de dicétones, facilement accessibles.

25. Utilisation du procédé selon les revendications 1 à 14, pour la synthèse des alkylpipérazines N-monosubstituées, en partant d'amines primaires facilement accessibles et de 3-azaglutardialdéhyde, dont la fonction amine est protégée de manière réversible, de préférence par un reste 2,2,2-trichloro-tert-butyloxycarbonyle.

26. Utilisation du procédé selon l'une ou plusieurs des revendications 1 à 14, pour la synthèse de composés N-hétérocycliques, en partant de composés nitrés ou nitrosés facilement accessibles.

27. Utilisation du procédé selon l'une ou plusieurs des revendications 1 à 14, pour la synthèse de médicaments, d'herbicides et d'insecticides et de leurs produits intermédiaires, dans lesquels, à côté des groupes aminés, se trouvent des groupes facilement réductibles, en particulier un groupe halogéno aromatique ou formyle.